# EUROPEAN PATENT APPLICATION

(11) **EP 1 352 964 A1**
(43) Date of publication of application: **15.10.2003**
(21) Application number: 00985832.5
(22) Date of filing: 22.12.2000
(51) Int. Cl.: C12P 7/42, C12P 11/00, C12P 13/02

(54) **PROCESS FOR PRODUCING SUBSTANCE BY USING MICROBIAL CATALYST**

(71) Applicant: NIPPON SODA CO., LTD., Chiyoda-ku, Tokyo 100-8165 (JP)
(72) Inventor: HAYAKAWA, Koichi, Takada, Odawara-shi, Kanagawa 250-0216 (JP); KOBAYASHI, Yoichi, Takada, Odawara-shi, Kanagawa 250-0216 (JP); YOKOYAMA, Masahiro, Takada, Odawara-shi, Kanagawa 250-0216 (JP)
(74) Representative: Wibbelmann, Jobst, Dr., Dipl.-Chem.
(86) International application number: JP0009138
(87) International publication number: WO02052027

(57) **Abstract**

An industrially advantageous process for producing a substance wherein the production speed of a reaction product in a reaction with the use of a microbial catalyst can be maintained at a constant level over a long period of time. In a reaction with the use of a microbial catalyst (for example, resting cells of an arthrobacter capable of converting a-hydroxynitriles into the corresponding a-hydroxy acid amides or a-hydroxy acid ammonium salts), the substance is produced at a constant speed by adding the substrate at a constant speed, regulating the concentration of the microbial catalyst in the liquid reaction mixture at a constant level and controlling the temperature so as to rise the reaction temperature at the point that the enzymatic activity of the microbial catalyst is lowered in the course of the reaction so as to maintain the reaction speed at a constant level.

## Description

### Technical Fields:

The present invention relates to processes for producing useful chemical substances, such as α-hydroxy acid ammonium salts, with microbial catalysts at fixed rates.

### Background Art:

A known method for keeping the production of a substance with a microbial catalyst at a fixed rate is to add a fresh microbial catalyst (Enzyme Catalysis in Organic Synthesis, VCH, 1995, Page 148).

Processes are also known for producing α-hydroxy acid ammonium salts or α-hydroxy acids from α-hydroxynitriles with microbial catalysts (Japanese Patents Laid-open Nos. Sho 58-15120, Sho 63-222696, Sho 64-10996, Hei 4-40897, Hei 4-40898 and Hei 10-507631).

### Disclosure of the Invention:

Keeping a production amount per reactor at a specified level is very advantageous in the industrial production of a substance in terms of stable shipment of the product and simple, easy reaction control. For a continuous reaction using a microbial catalyst, a reaction apparatus of which a reactor is combined with a separator is used. The microbial catalyst is continuously recovered for reuse and at the same time the product is continuously discharged as a separated filtrate. Generally a microbial catalyst gradually reduces its activity if used continuously. To keep the production rate at a specified level, a method of adding a fresh microbial catalyst has been adopted. The additional use of new microbial catalyst is not advantageous because of usually high production cost of microbial catalyst. Besides, a rise in the concentration of a microbial catalyst due to the addition of the catalyst results in increased load on the separator. Control of the reaction is a problem.

It is an object of the present invention to provide an industrially advantageous process for producing a substance wherein a product can be produced from a reaction using a microbial catalyst at a fixed rate over a long period of time.

The inventors studied in earnest to find answers for the above theme and fully analyzed changes of microbial catalyst activities with temperature. As a result, a method was found that a substrate is continuously added at a fixed rate while keeping the concentration of a microbial catalyst at a specified level only with the initial amount of the catalyst added and the reaction temperature is raised in order to produce a product at a fixed rate. Thus the present invention was completed.

The present invention relates to a process for producing a substance with a microbial catalyst in which a rise of the temperature of a reaction using a microbial catalyst is controlled to produce the substance at a fixed rate. It also relates to a process for producing a substance with a microbial catalyst in which a substrate of a microbial catalyst is added at a fixed rate and a reaction temperature rise is controlled while a reaction using a microbial catalyst is carried out in order to produce the substance at a fixed rate.

The present invention also relates to the said process for producing a substance with a microbial catalyst in which the concentration of the microbial catalyst is kept at a specified level in the reaction solution. It also relates to the said process for producing a substance with a microbial catalyst in which the microbial catalyst contained in the reaction solution discharged from the reactor is separated to recover and returned again to the reactor in order to keep the concentration of the microbial catalyst in the reaction solution at a specified level. It also relates to the said process for producing a substance with a microbial catalyst in which the temperature rise is in a range selected between 5 and 25°C. It also relates to the said process for producing a substance with a microbial catalyst in which the microbial catalyst is a microbial catalyst expressing nitrilase. It also relates to the said process for producing a substance with a microbial catalyst in which the microbial catalyst expressing nitrilase is a microorganism belonging to *Arthrobacter.*

The present invention also relates to the said process for producing a substance with a microbial catalyst in which the reaction using the microbial catalyst is a reaction that a nitrile compound is hydrolyzed by the catalytic action of the microorganism in an aqueous solvent to convert to an amide or a carboxylic acid or its ammonium salt In more detail, it also relates to the said process for producing a substance with a microbial catalyst in which the reaction using the microbial catalyst is a reaction that a nitrile compound represented by Formula RCN (wherein, R is hydrogen, optionally substituted alkyl having 1 to 6 carbons, optionally substituted alkenyl having 2 to 6 carbons, optionally substituted aryl or optionally substituted heterocyclic group) is hydrolyzed by the catalytic action of the microorganism in an aqueous solvent to convert to an amide represented by Formula RCONH₂ (wherein, R is as defined above) or a carboxylic acid represented by Formula RCOOH (wherein, R is as defined above) or its ammonium salt. It also relates to the said process for producing a substance with a microbial catalyst in which the reaction using the microbial catalyst is a reaction that an α-hydroxynitrile represented by Formula R'CH(OH)CN (wherein, R' is hydrogen, optionally substituted alkyl having 1 to 6 carbons, optionally substituted alkenyl having 2 to 6 carbons, optionally substituted alkoxy having 1 to 6 carbons, optionally substituted aryl, optionally substituted aryloxy or optionally substituted heterocyclic group) is hydrolyzed by the catalytic action of the microorganism in an aqueous solvent to convert to an α-hydroxy acid amide represented by Formula R'CH(OH)CONH₂ (wherein, R' is as defined above) or an α-hydroxy acid represented by Formula R'CH(OH)COOH (wherein, R' is as defined above) or its ammonium salt. It also relates to the said process for producing a substance with a microbial catalyst in which the reaction using the microbial catalyst is a reaction that an α-aminonitrile represented by Formula R"CH(NH₂)CN (wherein, R" is hydrogen, optionally substituted alkyl having 1 to 6 carbons, optionally substituted alkenyl having 2 to 6 carbons, optionally substituted alkoxy having 1 to 6 carbons, optionally substituted aryl, optionally substituted aryloxy or optionally substituted heterocyclic group) is hydrolyzed by the catalytic action of the microorganism in an aqueous solvent to convert to an α-amino acid amide represented by Formula R"CH(NH₂)CONH₂ (wherein, R" is as defined above) or an α-amino acid represented by Formula R''CH(NH₂)COOH (wherein, R" is as defined above) or its ammonium salt.

The processes of the present invention for the preparation of substances with microbial catalysts are characterized in that the substances are produced at fixed rates from reactions with microbial catalysts by means of controlling reaction temperature rises, preferably while adding substrates of the microbial catalysts at fixed rates and keeping the concentrations of the catalysts in the reaction solutions at specified levels.

Any microbial catalyst can be used in the present invention if it is a catalyst derived from a microorganism having an enzymatic activity such as that of hydrolase including nitrilase, oxidoreductase, transferase, isomerase, splitting enzyme and conjugating enzyme. Its examples include microorganism cells and treated products of microorganisms such as immobilized microorganisms, crude enzymes and immobilized enzymes. Either living or dead cells can be used if the microbial catalysts are microorganism cells or immobilized microorganisms.

A preferred microbial catalyst for use increases a reaction rate thanks to a temperature rise more than a decrease in the activity in the relation between the lowering rate of the enzymatic activity of the microbial catalyst and dependence of the reaction rate on temperature. Its actual examples include resting cells *of Arthrobacter* sp. NSSC104 (FERM P-15424), which can convert α-hydroxynitriles to corresponding α-hydroxy acid ammonium salts.

Examples of microorganisms able to be used as microbial catalysts include microorganisms belonging to genera of *Arthrobacter* other than the *afore-mentioned Arthrobacter* sp. NSSC104, *Caseobacter, Pseudomonas, Alcaligenes, Corynebacterium, Brevibacterfum, Nocardia, Rhodococcus, Gordona, Bacillius, Bacteridium, Micrococccus and Acinetobacter.*

There are no particular restrictions on compounds to be used as substrates in the present invention if they can be used for the reactions with microbial catalysts.

In the present invention, examples of substituents that Substituent R, R' or R" of Formula RCN, R' CH(OH)CN or R"CH(NH₂)CN may each have include hydroxyl, mercapto, carboxyl, carbamoyl, carbamidino, phenyl optionally substituted with hydroxyl or others, indolyl, imidazolyl, alkylthio having 1 to 6 carbons, alkenyl having 2 to 6 carbons, alkoxy having I to 6 carbons, aryloxy, and aralkyl such as benzyl.

Examples of the nitriles used in the present invention include nitrile compounds corresponding to various α-amino acids or their analogs of α-hydroxy acids.

Actual examples of the nitriles include acetonitrile, propionitrile, n-butyronitrile, i-butyronitrile, acrylonitrile, methacrylonitrile, benzonitrile, cyanopyridine, malononitrile, succinonitrile, fumaronitrile, lactonitrile, 3-hydroxypropionitrile, aminoacetonitrile, 2-aminopropionitrile, 3-aminopropionitrile, 2-aminophenylpropionitrile, 2-methylthiobutyronitrile, phenylglycinonitrile, p-hydroxyphenylglycinonitrile, o-hydroxyphenylglycinonitrile, 2-amino-3-hydroxypropionitrile, 2-amino-3-phenylpropionitrile, 2-amino-4-phenylbutyronitrile, 2-amino-3-methylvaleronitrile, 2-amino-4-methylvaleronitrile, 2-amino-3-butyronitrile, 2-amino-4-methylthiobutyronitrile, lactonitrile, mandelonitrile and 2-hydroxy-4-methylthiobutyronitrile.

A reaction with a microbial catalyst in the present invention is preferably carried out using resting cells in an aqueous solvent. In the case of a reaction with resting cells, a concentration of the microbial catalyst in the reaction solution can be arbitrarily determined depending on the type of a product produced and the characteristics of the microbial catalyst used. It is usually within a range of 0.1 to 10.0% by weight as dried cells. It is preferable to keep the concentration of the microbial catalyst in the reaction solution at a specified level from the viewpoint of substance production costs and operations. For example, a microbial catalyst in a reaction solution discharged from a reactor is separated to recover, and the recovered catalyst is returned again to the reactor so as to keep the microbial catalyst at a specified level in concentration in the reaction solution. Production costs of microbial catalysts are usually high. Besides, a rise in the concentration of a microbial catalyst in a reactor due to the addition of the catalyst results in increased load on a separator, and control of the reaction with the microbial catalyst becomes complicated.

The pH of a reaction solution with a microbial catalyst differs depending on a type of a microbial catalyst used. It is usually selected from a pH range of 5 to 10. An addition of an acid or alkali to a reaction solution can keep the pH of the reaction solution at a specified level. It is not however always the case to keep pH at a fixed level during a reaction, depending on a microbial catalyst used.

In the present invention, "control of reaction temperature rise" refers to that the enzymatic activity of a microbial catalyst is controlled to be unvarying by increasing the reaction temperature. The control of the reaction temperature rise makes it possible to produce a substance at a fixed rate. Conditions for controlling the temperature rise can be determined by analyses of a lowering rate of the enzymatic activity and dependence of a reaction rate on temperature. Measurements of a concentration of a substrate or product in a reaction solution will give actual control conditions. A rise of reaction temperature can be arbitrarily determined in consideration of the type and characteristics of a microbial catalyst used and a period of time to keep the production rate at a specified level. It is usually between 5 and 25'C, preferably 10 and 20°C. A reaction with a microbial catalyst is carried out in a specified temperature range selected from 0 to 50°C, preferably 20 to 40°C.

In the present invention, "production of a substance at a fixed rate" refers to that a substance is produced at a practically fixed reaction rate, while practically keeping the production of a product per unit volume of the reaction solution at a fixed rate. This makes it possible to maintain an amount of the product produced per reactor at a specified level when the substance is produced at an industrial scale and is very advantageous in terms of stable shipping of the product and simple, easy reaction control. In an enzymatic reaction, the reaction rate becomes closer to the maximum reaction rate as the concentration of a material (substrate) becomes higher in concentration. The higher the concentration of the material (substrate), the more it remains in the reaction solution. Therefore, it is preferable to determine a concentration of a material (substrate) from the viewpoint of productivity such as yield of a product

To describe a process of the present invention for producing a substance with a microbial catalyst, a method of using a reaction apparatus consisting of a stirred tank reactor combined with a separator is taken as an example. A microbial catalyst is fed into an aqueous solvent in a reactor so as to be the catalyst concentration at a specified level. Then, an addition of a material substrate starts at a fixed rate. Immediately when the substrate is added, the reaction starts. When the product is accumulated in the reaction solution to the target level, a separator starts operating to separate the product from the microbial catalyst The whole amount of the separated and recovered catalyst is continuously returned to the reactor. Meanwhile, the product in the reaction solution is discharged continuously from the system at a fixed rate. When the separation operation starts, it is preferable to continue the addition of the substrate at the fixed rate and at the same time to add water or an aqueous solvent consisting of an aqueous solution with an organic solvent for adjusting the concentration in order to keep the concentration of the product accumulated at the specified level.

In the process of the continuous reaction with the microbial catalyst, the activity of the catalyst in the reaction solution lowers at a certain rate that depends on reaction conditions. This activity decrease is made up for by raising the reaction temperature. It is necessary to control the reaction temperature rise for the production of a substance at a fixed rate. The reaction temperature rise is controlled by that, after the catalyst is separated, the concentrations of the remaining substrate and product accumulated in the solution containing the product are measured continuously or intermittently by a device, such as high-performance liquid chromatography, and the temperature control system of the reactor is programmed so that the concentrations are always at the specified levels. A period during which the reaction rate is kept constant is a period that the decrease of the activity of the microbial catalyst can be made up for by raising the reaction temperature to increase the reaction rate. It differs depending on the characteristics of a microbial catalyst used and is usually about 10 days to 3 months.

Examples of the afore-mentioned separator used for separating a product from a microbial catalyst include membrane separators and centrifugal separators. When the product is dissolved in the aqueous solvent, a centrifugal separator or a membrane separator such as that with a microfiltration or ultrafiltration membrane can easily separate the product from the microbial catalyst. In case the product is not dissolved in the aqueous solvent, a filtration membrane having a pore size in the middle of the particle sizes of the microbial catalyst and the product can separate them. A plug flow reactor, such as a membrane-type reactor of which a stirred tank reactor is integrated with a separator, can be used as a reaction apparatus, in addition to the said apparatus of a stirred tank reactor combined with a separator.

The product is separated from the solution containing the product by, for example, evaporating the aqueous solvent to concentrate when the product is dissolved in the aqueous solvent, or by a solid-liquid separator or another similar device when the product is not dissolved in the aqueous solvent. If the product needs purification, usual purification procedures, such as extraction with organic solvents, distillation or recrystallization, can be employed.

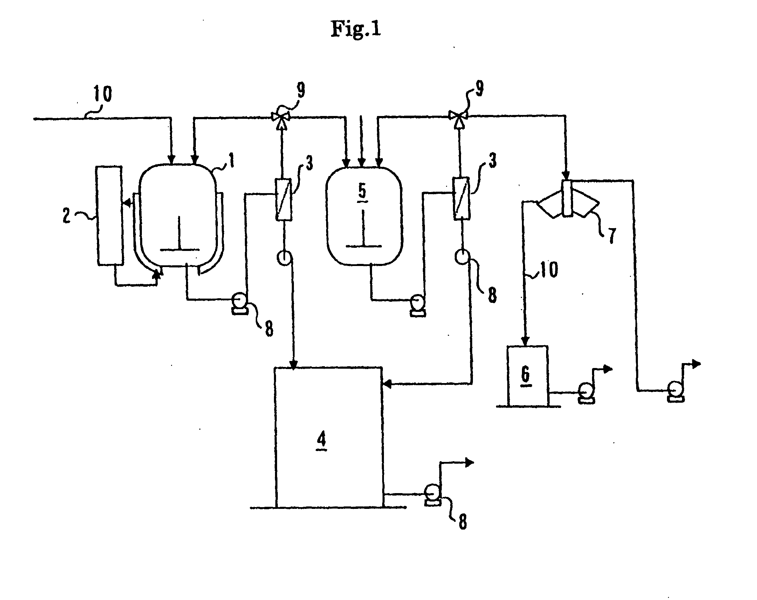

An example of a substance production plant is shown in Figure 1 when a process of the present invention for producing a substance with a microbial catalyst is applied for an actual production. The plant consists of Reactor 1, Temperature Control System 2 attached to Reactor 1, Membrane Separator 3, Filtrate Storage Tank 4, Washing Tank 5, Waste Cells Storage Tank 6, Centrifugal Separator 7, a few Pumps 8, Three-way Valve 9, and Pipes 10 that connect these. As described above, a continuous reaction is carried out using Reactor 1 of which a temperature rise can be controlled by Temperature Control System 2, together with Membrane Separator 3. The microbial catalyst separated from the filtrate by Membrane Separator 3 is recovered and returned to Reactor 1. Meanwhile, the filtrate is recovered into Filtrate Storage Tank 4. Upon the completion of the reaction, the whole reaction solution is transferred into Washing Tank 5. The microbial catalyst in the reaction solution is separated from the filtrate by Membrane Separator 3 and washed with water in Washing Tank 5. The washing water is collected in Filtrate Storage Tank 4, same as the said filtrate. The waste cells after washed are dehydrated in Centrifugal Separator 7 and sent to Waste Cells Storage Tank 6.

### Brief Descriptions of the Figures:

Figure 1 shows a schematic diagram of a substance production plant when a process of the present invention for producing a substance with a microbial catalyst is applied for actual production.
Figure 2 shows changes with time of the production rate of 2-hydroxy-4-methylthiobutyric acid ammonium salt and the reaction temperature in Example 1 of the present invention.

### Best Form to Implement the Invention:

The present invention is described in more detail in reference to the examples, but not limited to these examples. The yield of the product was determined by a measurement of the weight of the product obtained or volume of the aqueous solvent solution containing the product and further measuring the concentrations of the remaining substrate and the product by such a means as high-performance liquid chromatography or gas chromatography. An amount of ammonia was determined by a UV absorbance method using NADH - glutamic acid dihydrogenase (Methods of Enzymatic Analysis, Bergmeyer H. U. 3rd edition, Vol. 8, Pages 454 to 461).

### Example 1

Into a 300-ml stirred tank reactor equipped with an ultrafiltration (UF) membrane separator, an automatic dilution device, an on-line high-performance liquid chromatography with an auto-injector and a pH controller was placed 245.4g (17.2g when dried) of 35% wet cells *ofArthrobacter* sp. NSSC104 that was cultured beforehand to use as a microbial catalyst, and further diluted with 29.8 ml of water. Into the reactor was continuously added 2-hydroxy-4-methylthiobutyronitrile as a substrate at a rate of 0.227g/minute. The reaction started at a reaction temperature of 21°C. When a total of 51.8g of the substrate was added, a continuous addition of water started at a rate of 0.945g/ minute.

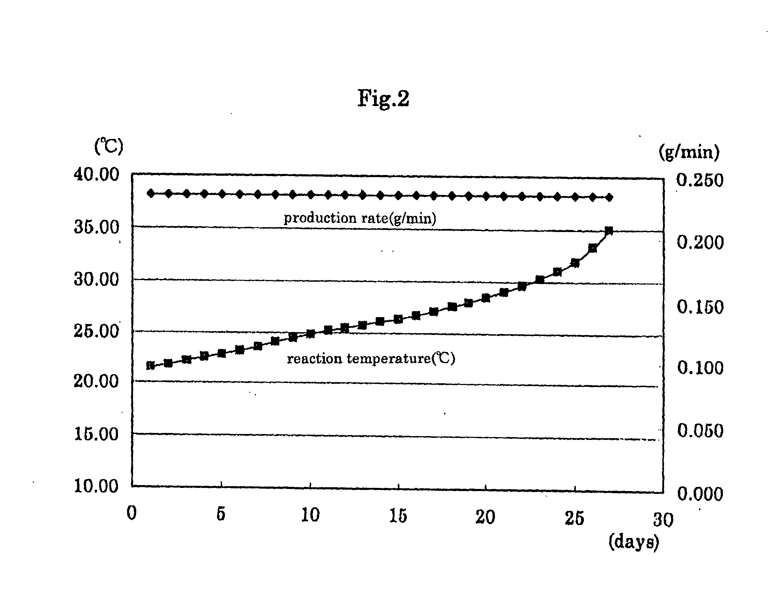

While keeping the continuous addition of 2-hydroxy-4-methylthiobutyronitrile at the rate of 0.227g/minute, the reaction solution was transferred from the reactor to the membrane separator where the microbial catalyst in the reaction solution was separated by the membrane separator and recovered to return to the reactor, and at the same time the filtrate filtered out from the membrane separator was discharged at a rate of 1.173g/minute. Throughout the reaction, 28% aqueous ammonia was added to keep the pH to 7.0. To keep the product, 2-hydroxy-4-methylthiobuyric acid ammonium salt, being produced at a fixed rate while the continuous reaction was carried out, the concentration of 2-hydroxy-4-methylthiobutyronitrile in the reaction solution was measured by the on-line high-performance liquid chromatography and the reaction temperature was increased whenever the concentration exceeded about 0.2% by weight in order to control the concentration to be about 0.2% by weight. Then, the filtrate filtered out through the membrane and containing about 22% by weight of 2-hydroxy-4-methylthiobutyric acid ammonium salt was discharged at the said fixed rate. A total amount of 473 kg of the filtrate filtered out through the membrane was obtained from the continuous reaction for 27 days. The reaction temperature was 35°C, up by 14°C, on the final day. Changes of the production rate and reaction temperature with time are shown in Figure 2.

When the 27-day continuous reaction was completed, the solutions remaining in the reactor and reaction piping were concentrated and washed through the membrane to recover separately 534.5g of filtrate filtered out through the membrane. Analyses by the high-performance liquid chromatography of the remaining amounts of the substrate, 2-hydroxy-4-methylthiobutyronitrile, in the membrane-filtered filtrate obtained during the continuous reaction and in the filtrate obtained from the remaining solutions washed through the membrane and recovered separately, showed 0.25% by weight and 0.12% by weight, respectively. Similar analyses of the product, 2-hydroxy-4-methylthiobutyric acid, showed 20.00% by weight and 9.35% by weight, respectively, and 0.07% by weight and 0.03% by weight, respectively, for a byproduct, 2-hydroxy-4-methylthiobutyric acid amide. The substrate used, 2-hydroxy-4-methylthiobutyronitrile, was 95.0% by weight in purity. Calculations of the yield of the product in mole and remaining rate of the substrate resulted in 95.0% and 1.35%, respectively. The amounts of ammonia in the filtrates from the reaction solution and from the remaining solutions in the reaction apparatus, measured by an enzymatic method, were 2.28% by weight and 1.07% by weight, respectively.

These filtrates were put together and washed with toluene of an amount one-tenth of the total of the filtrates. The aqueous layer was concentrated to 13.5 kg by a rotary evaporator at 50 to 70 mmHg in a bath set to 50°C. Analyses of the concentrated solution by the high-performance liquid chromatography showed 0.06% by weight of the substrate, 2-hydroxy-4-methyithiobutyronitrile, remaining and 70.44% by weight of the product, 2-hydroxyl--methylthiobutyrnic acid, contained, with 0.23% by weight of 2-hydroxy-4-methyithiobutyric acid amide and 0.15% by weight of a chain dimmer of 2-molecule condensed ester of Z-hydroxy-4-methylthiobutyric acid as byproducts. Calculations of the yield of the product in mole and remaining rate of the substrate after the filtrates were concentrated resulted in 95% and 0.09%, respectively. A measurement by an enzymatic method found 7.18% by weight of ammonia. These figures confirmed the decomposition and distillation of the substrate, 2-hydroxy-4-methylthiobutyronitrile, during the concentration operation.

### Applicability in Industry:

The present invention makes it possible to recover the whole amount of a microbial catalyst added at the beginning of the reaction without newly adding an expensive microbial catalyst, and to maintain the production of a product per reactor at a fixed rate by controlling a reaction temperature rise. Thanks to the above advantages, an expensive microbial catalyst is utilized efficiently and a useful substance is produced in high yield as well as an industrially advantageous, stable production is materialized.

## Claims

1. A process for producing a substance with a microbial catalyst in which a rise of the temperature of a reaction using a microbial catalyst is controlled to produce the substance at a fixed rate.

2. A process for producing a substance with a microbial catalyst in which a substrate of the microbial catalyst is added at a fixed rate and a reaction temperature rise is controlled while a reaction using a microbial catalyst is carried out in order to produce the substance at a fixed rate.

3. A process for producing a substance with a microbial catalyst according to Claim 1 or 2 in which the concentration of the microbial catalyst is kept at a specified level in the reaction solution.

4. A process for producing a substance with a microbial catalyst according to Claim 3 in which the microbial catalyst contained in the reaction solution discharged from the reactor is separated to recover and returned again to the reactor in order to keep the concentration of the microbial catalyst in the reaction solution at a specified level.

5. A process for producing a substance with a microbial catalyst according to one of Claims 1 to 4 in which the temperature rise is in a range selected between 5 and 25°C.

6. A process for producing a substance with a microbial catalyst according to one of Claims 1 to 5 in which the microbial catalyst is a microbial catalyst expressing nitrilase.

7. A process for producing a substance with a microbial catalyst according to Claim 6 in which the microbial catalyst expressing nitrilase is a microorganism belonging to *Arthrobacter.*

8. A process for producing a substance with a microbial catalyst according to one of Claims 1 to 7 in which the reaction using the microbial catalyst is a reaction that a nitrile compound represented by Formula RCN (wherein, R is hydrogen, optionally substituted alkyl having 1 to 6 carbons, optionally substituted alkenyl having 2 to 6 carbons, optionally substituted aryl or optionally substituted heterocyclic group) is hydrolyzed by the catalytic action of the microorganism in an aqueous solvent to convert to an amide represented by Formula RCONH₂ (wherein, R is as defined above) or a carboxylic acid represented by Formula RCOOH (wherein, R is as defined above) or its ammonium salt

9. A process for producing a substance with a microbial catalyst according to one of Claims 1 to 7 in which the reaction using the microbial catalyst is a reaction that an α-hydroxynitrile represented by Formula R'CH(OH)CN (wherein, R' is hydrogen, optionally substituted alkyl having 1 to 6 carbons, optionally substituted alkenyl having 2 to 6 carbons, optionally substituted alkoxy having 1 to 6 carbons, optionally substituted aryl, optionally substituted aryloxy or optionally substituted heterocyclic group) is hydrolyzed by the catalytic action of the microorganism in an aqueous solvent to convert to an α-hydroxy acid amide represented by Formula R'CH(OH)CONH₂ (wherein, R' is as defined above) or an α-hydroxy acid represented by Formula R'CH(OH)COOH (wherein, R' is as defined above) or its ammonium salt.

10. A process for producing a substance with a microbial catalyst according to one of Claims 1 to 7 in which the reaction using the microbial catalyst is a reaction that an α-aminonitrile represented by Formula R"CH(NH₂)CN (wherein, R" is hydrogen, optionally substituted alkyl having 1 to 6 carbons, optionally substituted alkenyl having 2 to 6 carbons, optionally substituted alkoxy having 1 to 6 carbons, optionally substituted aryl, optionally substituted aryloxy or optionally substituted heterocyclic group) is hydrolyzed by the catalytic action of the microorganism in an aqueous solvent to convert to an α-amino acid amide represented by Formula R"CH(NH₂)CONH₂ (wherein, R" is as defined above) or an α-amino acid represented by Formula R"CH(NH₂)COOH (wherein, R" is as defined above) or its ammonium salt.
